# EUROPÄISCHE PATENTSCHRIFT

(11) **EP 1 568 780 B1**
(45) Veröffentlichungstag und Bekanntmachung des Hinweises auf die Patenterteilung: **25.04.2007**
(21) Anmeldenummer: 05002798.6
(22) Anmeldetag: 10.02.2005
(51) Int. Cl.: C12P 7/02, C12P 7/04, C12P 7/62

(54) **Verfahren zur enantioselektiven Reduktion von Ketoverbindungen durch Enzyme**
Enzymatic method for the enantioselective reduction of keto compounds
Procédé enzymatique de reduction enantioselective de composes cetoniques

(30) Priorität: 12.02.2004 DE 102004007029
(43) Veröffentlichungstag der Anmeldung: 31.08.2005
(73) Patentinhaber: Consortium für elektrochemische Industrie GmbH, 81379 München (DE)
(72) Erfinder: Peschko, Christian, Dr., 81543 München (DE); Stohrer, Jürgen, Dr., 82049 Pullach (DE)
(74) Vertreter: Fränkel, Robert

(56) Entgegenhaltungen:
- WO-A-96/11256
- WO-A-02/064579
- WO-A-02/086126
- DE-A1- 19 610 984
- DE-C1- 4 014 573
- FILHO ET AL: "Is logP a convenient criterion to guide the choice of solvents for biphasic enzymatic reactions?" ANGEW. CHEM., Bd. 115, 2003, Seiten 3101-3104, XP002328868

## Beschreibung

Die vorliegende Erfindung betrifft ein enzymatisches Verfahren zur enantioselektiven Reduktion von organischen Ketoverbindungen zu den entsprechenden chiralen Hydroxyverbindungen.

Optisch aktive Hydroxyverbindungen sind wertvolle Synthesebausteine zur Herstellung wichtiger Verbindungen mit pharmakologischer Wirkung und anderen wertvollen Eigenschaften. Diese Verbindungen sind durch klassische chemische Verfahren oft nur schwer herstellbar und die erforderlichen optischen Reinheiten für Anwendungen im pharmazeutischen oder agrochemischen Bereich auf diesem Wege nur schwer zu erreichen. Daher werden zur Herstellung chiraler Verbindungen im zunehmenden Maße biotechnologische Verfahren angewendet, wobei die stereoselektive Reaktion von ganzen Mikroorganismen oder mit vollständig oder teilweise gereinigten, isolierten Enzymen durchgeführt wird.

Dehydrogenasen und insbesondere Alkohol-Dehydrogenasen (ADH) sind wertvolle Katalysatoren zur Gewinnung chiraler Produkte durch stereoselektive Reduktion von organischen Ketoverbindungen zu den entsprechenden chiralen Alkoholen. Bekannt sind im Wesentlichen entsprechende Enzyme aus Hefe, Pferdeleber oder Thermoanaerobium brockii. Diese Enzyme benötigen als Coenzym NADH (Nikotinamidadenindinukleotid) oder NADPH (Nikotinamidadenindinukleotidphosphat). Weitere bekannte Alkoholdehydrogenasen sind beispielsweise eine *(S)*-spezifische Alkohol-Dehydrogenase aus Rhodococcus erythropolis oder eine (R)-spezifische Alkohol-Dehydrogenase aus der Gattung Lactobacillus. Beide Enzymtypen haben ein breites Substratspektrum an Ketoverbindungen und weisen eine hohe Enantioselektivität auf. Die Alkohol-Dehydrogenasen aus Lactobacillus kefir (DE 40 14 573 C1) und Lactobacillus brevis (DE 196 10 984 A1) eignen sich insbesondere zur Gewinnung von chiralen (R)-Alkoholen.

Aus dem Stand der Technik sind Verfahren zur stereoselektiven Reduktion organischer Ketoverbindungen zu den entsprechenden chiralen Alkoholen durch Alkohol-Dehydrogenasen, bei denen die Ketoverbindung mit Wasser, Enzym, Coenzym, sowie einem zur Regeneration des Enzymsystems benötigten Reduktionsmittel in Kontakt gebracht werden, bekannt (z.B. WO 96 11256).

Nachteil solcher enzymkatalysierten Verfahren sind die hohen Enzymkosten, die durch hohen spezifischen Enzymverbrauch verursacht werden. Im Stand der Technik wird daher meist mit sehr geringen Enzymmengen gearbeitet. Die nach dieser Verfahrensart erzielten Raum-Zeit-Ausbeuten sind durchgängig gering und erfahren wegen hoher Prozesskosten keine wirtschaftliche bzw. großtechnische Umsetzung.

So wird in WO 02/064579 A1 ein Verfahren beschrieben, bei dem für die ADH-LB-katalysierte Reduktion von Alkinonen beispielsweise ein Enzymeinsatz von 0.5 Units /mL (U/mL) verwendet wird und damit Raum-Zeit-Leistungen von nur etwa 15 mol/m³d bei einem Enzymverbrauch von etwa 60 kU pro mol Produkt erzielt werden. Unter der Bezeichnung mol/m³d versteht man die bei einem Herstellungsprozess pro Kubikmeter Ansatzvolumen und pro Tag gewonnene Produktmenge in mol.

Ein in DE 196 10 984 A1 beschriebenes Verfahren zur Reduktion von Ketonen mit ADH-LB erzielt mit einem Enzymeinsatz von 4.5 U/mL eine Raum-Zeit-Leistung von 4.75 mol/m³d bei einem Enzymverbrauch von etwa 950 kU pro mol Produkt.

Wegen der oft unzureichenden Löslichkeit der organischen Substrate im wässrigen Milieu werden auch Zweiphasen-Systeme eingesetzt, die neben Wasser eine weitere organische Phase enthalten. Die Gegenwart einer organischen Phase wirkt sich aber in unterschiedlichem Umfang destabilisierend auf die Enzymaktivität aus (M. V. Filho, T. Stillger, M. Müller, A. Liese, C. Wandrey, Angew. Chem., **2003,** 115, 3101-3104). Die Gegenwart einer organischen Phase kann aber auch zu einer erhöhten Aktivität des Enzyms führen (M. V. Filho, T. Stillger, M. Müller, A. Liese, C. Wandrey, Angew. Chem., **2003**, 115, 3101-3104).

Verfahren mit zweiphasigen Systemen weisen teilweise verbesserte Raum-Zeit-Leistungen auf. Der Enzymverbrauch ist in der Regel aber ebenfalls hoch. Verfahren dieser Art sind daher ebenso wirtschaftlich und großtechnisch nur begrenzt einsetzbar.

Aus WO 02/086126 A2 ist ein Verfahren für die ADH-LB-katalysierte Reduktion eines β-Ketoesters im zweiphasigen System bekannt, für das bei Raum-Zeit-Leistungen von 200 bis 350 Mol/m³d zwischen 22.5 und 27.4kU Enzym pro Mol Produkt verbraucht werden.

Aufgabe der Erfindung ist es daher, ein Verfahren zur enzymkatalysierten Herstellung von chiralen Alkoholen mit hoher Raum-Zeit-Leistung bei zugleich niedrigem Enzymverbrauch bereitzustellen.

Diese Aufgabe wird gelöst in einem Verfahren zur enzymkatalysierten Reduktion von Ketoverbindungen unter Verwendung eines Reaktionsmediums, enthaltend Alkohol-Dehydrogenase, Wasser, Coenzym, Reduktionsmittel, aus dem nach der Reduktion der Ketoverbindung in einem ersten Schritt mit einem organischen Lösemittel die Reaktionsprodukte in einem zweiten Schritt extrahiert werden. Nach der Extraktion wird die organische Phase enthaltend die Reaktionsprodukte abgetrennt und das wässrige Reaktionsmedium erneut verwendet und das Verfahren wiederholt.

Gegenstand der Erfindung ist ein Verfahren zur Herstellung chiraler sekundäre Alkohole enthaltend die Schritte
a) Reduktion einer Ketoverbindungen in einem wässerigen Reaktionsmedium, enthaltend Wasser, Reduktionsmittel, Alkoholdehydrogenase (ADH) und Coenzym unter vermindertem Druck und Entfernung leicht flüchtiger Bestandsteile aus dem Reaktionssystem,
b) Extraktion des gebildeten sekundären Alkohols mittels einer weitere Phase, enthaltend ein nicht mit Wasser mischbares organisches Lösemittel unter Verwirbelung und/oder Durchmischung der wässerigen und organischen Phasen,
c) Abtrennung der zur Extraktion verwendeten Phase und Wiederverwendung des wässrigen Reaktionsmediums in Schritt a).

Das erfindungsgemäße Verfahren zeichnet sich durch hohe Raum-Zeit-Leistungen bei zugleich niedrigem Enzymverbrauch, Enantiomerenreinheiten von bis zu 99.9% bezüglich der hergestellten chiralen Hydroxyverbindungen und chemische Ausbeuten von bis zu >99% bezogen auf die eingesetzte Menge an Ketoverbindung aus. Die ständige Gegenwart einer potentiell enzymdesaktivierenden organischen Lösemittelphase, wie dies in den bekannten 2-Phasen-Verfahren der Fall ist, wird vermieden. Ferner ist das erfindungsgemäße Verfahren sehr einfach apparativ und somit großtechnisch umsetzbar.

Die eigentliche Reduktion gemäß Schritt a) wird dabei in einem wässrigen System durchgeführt, ohne dass eine weitere Phase, die lediglich der Extraktion dient und ein nicht mit Wasser mischbares organisches Lösemittel enthält, gleichzeitig anwesend ist und/oder zugesetzt wird. Auf den gezielten Zusatz einer ständig im System anwesenden organischen Extraktionsphase wird demnach in dem erfindungsgemäßen Verfahren verzichtet.

Gleichwohl ist nicht ausgeschlossen, dass sich am Anfang oder im Verlauf der Reduktion eine von der wässrigen Phase (Reaktionsmedium) getrennte weitere Phase ausbildet, die gegebenenfalls aus einer wenn auch nur anfänglich bestehenden Nichtmischbarkeit des zu reduzierenden Substrats (Ketoverbindung) mit dem wässrigen Reaktionsmedium resultiert.

Als Edukte können allgemein Ketone verwendet werden, bevorzugt solche mit 3 bis 40 C-Atomen.

In einer bevorzugten Ausführungsform des erfindungsgemäßen Verfahrens werden prochirale Ketone der allgemeinen Formel (I)

R¹-C(O)-R² (I)

eingesetzt, wobei
**R¹** und **R²** unabhängig voneinander ausgewählt werden aus der Gruppe enthaltend C₁-C₂₀-Alkyl, C₃-C₂₀-Cycloalkyl, C₅-C₂₀-Aryl, C₁-C₂₀-Heteroaryl, C₂-C₂₀-Alkenyl, C₅-C₂₀-Aralkyl, C₅-C₂₀-Alkylaryl oder R¹ und R² zusammen einen Ring bilden können
und **R¹** und **R²** gegebenenfalls unabhängig voneinander mit einem oder mehreren Resten Z substituiert sein können, wobei
**Z** ausgewählt wird aus der Gruppe enthaltend Fluor, Chlor, Brom, Iod, -CN, -NO₂, -NO, -NR³OR³, -CHO, -SO₃H, -COOH oder -R³ und
**R³** für Wasserstoff steht oder die Bedeutung von **R¹** haben kann und
in R¹ und R² gegebenenfalls unabhängig voneinander eine oder mehrere Methylengruppen durch gleiche oder verschiedene Gruppen **Y** ersetzt sein können, wobei
**Y** ausgewählt wird aus der Gruppe enthaltend -CR³=CR³-, -C=C-, -C (O) -, -C(O)O-, -OC (O) -, -C (O) OC (O) -, -O-, -O-O-, -CR³=N-, -C(O)-NR³-, -N=N-, -NR³-NR³-, -NR³-O-, -NR³-, -P(O)(OR³)O-, -OP(O)(R³)O-, -P(R³)-, -P(O)(R³)-, -S-, -S-S-, -S(O)-, -S(O)₂-, -S(O)NR³-, -S(O)(OR³)O-, -Si(R³)₂-, -Si(R³)₂O-, -Si(R³)(OR³)-, -OSi(R³)₂O-, -OSi(R³)₂-oder -Si(R³)₂OSi(R³)₂-.

Bevorzugte C₅-C₂₀-Aryl oder C₁-C₂₀-Heteroaryl-Reste für R¹ und R² werden insbesondere ausgewählt aus der Gruppe enthaltend Phenyl, Naphthyl, Indolyl, Benzofuranyl, Thiophenyl, Pyrrolyl, Pyridinyl, Imidazolyl, Oxazolyl, Isoxazolyl, Furanyl oder Thiazolyl.

Die Verbindungen der allgemeinen Formel (I) können im Allgemeinen auch als Salze eingesetzt werden.

Besonders bevorzugte Verbindungen der allgemeinen Formel (I) werden ausgewählt aus der Klasse der β-Ketoester, α-Ketoester, γ-Ketoester, β-Diketone, γ-Diketone, δ-Diketone, β-Halogenketone, α-Halogenketone, αα-Dihalogenketone, α-Alkoxyketone, α-Acyloxyketone, β-Alkoxyketone, α-Alkinylketone, Alkenylketone, α-Diketone, αα-Dialkoxyketone, Arylketone, Hetarylketone.

Insbesondere eignen sich als Verbindungen der allgemeinen Formel (I) 3-Oxo-butansäuremethylester, 3-Oxobutansäureethylester, 4-Chlor-3-oxo-butansäuremethylester, 4-Chlor-3-oxo-butansäureethylester, 3-Oxo-pentansäuremethylester, 3-Oxo-pentansäureethylester, 1-Chlor-propan-2-on, 1,1-Dichlor-propan-2-on, 3-Oxo-butan-2-on, 2,6-Dimethyl-hexan-3,5-dion, 2,7-Dimethyl-hexan-3,6-dion, 2,4-Hexandion, 2,4-Pentandion, 3-Oxo-butansäuretertbutylester, 2,5-Hexandion, 4-Trimethylsilyl-3-butin-2-on, 4-Triisopropylsilyl-3-butin-2-on, 1-Chlor-4-trimethylsilyl-3-butin-2-on, 1-Chlor-4-triisopropylsilyl-3-butin-2-on oder 1-Chlor-butin-2-on, 1-Chlorbutan-3-on, Butanon, Pentan-2-on, Hexan-2-on, Heptan-2-on, Octan-2-on, 3-Penten-2-on, 1-Acetoxypropan-2-on, Cyclopent-2-en-1-on, 3-Oxotetradecansäuremethylester, 3-Oxododecansäuremethylester, 2-Oxo-propionsäureethylester, 1,4-Dichlorbutanon, Acetophenon, 3-Methylbutanon, 1-Benzyloxy-propan-2-on oder 2-Methylcyclopentanon.

Die Verbindungen der allgemeinen Formel (I) werden im erfindungsgemäßen Verfahren in einer Menge von 1% bis 50% bezogen auf das Gesamtvolumen eines jeden Reaktionsansatzes eingesetzt, bevorzugt von 3% bis 25%, insbesondere von 5% bis 15%.

Die Reaktionsmischung sollte generell einen pH-Wert von 5 bis 10 haben, bevorzugt von 6 bis 9.

Die wässrige Phase (Reaktionsmedium) enthält bevorzugt einen Puffer, insbesondere einen Kaliumphosphat/Kaliumhydrogenphosphat-, Tris(hydroxymethyl)aminomethan/ HCl-oder Triethanolamin/ HCl-Puffer mit einem pH-Wert von 5 bis 10, vorzugsweise einem pH-Wert von 6 bis 9. Die Pufferkonzentration sollte von 5 mM bis 150 mM betragen.

Zusätzlich kann die wässrige Phase auch Magnesiumionen enthalten, beispielsweise in Form von zugesetztem MgCl₂ in einer Konzentration von 0.2 mM bis 10 mM, bevorzugt 0.5 mM bis 2 mM bezogen auf die eingesetzte Wassermenge. Daneben kann die wässrige Phase weitere Salze wie beispielsweise NaCl, sowie weitere Zusätze wie beispielsweise Dimethylsulfoxid, Glycerin, Glycol, Ethylenglycol, Sorbitol, Mannitol oder Zucker enthalten.

Als Coenzym können beispielsweise NADP, NADPH, NAD, NADH oder deren Salze eingesetzt werden. Die Konzentration an Coenzym in der wässrigen Phase beträgt bevorzugt von 0.01 mM bis 0.25 mM, besonders bevorzugt von 0.02 mM bis 0.1 mM.

Als Reduktionsmittel wird der wässrigen Phase im Allgemeinen ein sekundärer Alkohol, bevorzugt Isopropanol zugefügt. Die bei jedem Ansatz zugesetzte Alkoholmenge beträgt 1% bis 60% bezogen auf das Gesamtvolumen des Ansatzes, bevorzugt 1% bis 50%, insbesondere 1 bis 30%, besonders bevorzugt 4% bis 15%.

Als Reduktionsmittel kann auch Ameisensäure oder die Salze der Ameisensäure, insbesondere Natriumformiat zugesetzt werden, was gegebenenfalls die Zugabe zusätzlicher Stoffe wie beispielsweise Formiatdehydrogenase (FDH) erforderlich machen kann.

Geeignete Alkohol-Dehydrogenasen stammen beispielsweise aus Hefe, Pferdeleber oder Rhodococcus erythropolis, wobei diese Enzyme als Coenzym NADH benötigen, oder aus Thermoanaerobium spec., Lactobacillus kefir oder Lactobacillus brevis, wobei diese Enzyme als Coenzym NADPH benötigen.

Die Alkohol-Dehydrogenase kann im erfindungsgemäßen Verfahren entweder vollständig gereinigt oder teilweise gereinigt eingesetzt werden oder in Zellen enthaltend verwendet werden. Die eingesetzten Zellen können dabei nativ, permeabilisiert oder lysiert vorliegen.

Die Volumenaktivität der eingesetzten Alkohol-Dehydrogenase beträgt von 100 Units/ml (U/ml) bis 5 000 U/ml, bevorzugt etwa 1 000 U/ml. In der wässrigen Phase, die nach Produktextraktion wieder eingesetzt wird, stehen zur Umsetzung von je 1 kg Verbindung der allgemeinen Formel (I) bevorzugt 50 000 bis 700 000 U Alkohol-Dehydrogenase (ADH) zur Verfügung. Besonders bevorzugt wird die ADH in der wässrigen Phase mit mehr als 15 U/ml eingesetzt.

Die Temperatur des Reaktionsgemisches beträgt bevorzugt von 0°C bis 60°C, besonders bevorzugt von 20°C bis 40°C.

Im Laufe der Reaktion kann weiteres Reduktionsmittel oder Edukt insbesondere in Form von Ketonen der allgemeinen Formel (I) zugegeben werden.

Die Reaktion wird unter vermindertem Druck durchgeführt.

Je nach Art und Menge der eingesetzten Alkohol-Dehydrogenase und der eingesetzten Verbindung der allgemeinen Formel (I) beträgt die Reaktionszeit zwischen 30min und 50h, bevorzugt 1h bis 20h.

Die Extraktion mit der nicht mit Wasser mischbaren, ein organisches Lösemittel enthaltenden Extraktionsphase kann dabei diskontinuierlich (batchweise) oder kontinuierlich erfolgen. In den möglichen Ausführungsformen für den Extraktionsschritt kann die Extraktion das gesamte (diskontinuierlich) oder einen Teil des wässrigen Reaktionsmediums - insbesondere bei der kontinuierlichen Extraktion - umfassen.

Die wässrige Phase (Reaktionsmedium), enthaltend das gewünschte Reaktionsprodukt, wird mit einer zweiten Phase, enthaltend ein nicht mit Wasser mischbares organisches Lösemittel in einer dem Fachmann bekannten Weise diskontinuierlich oder bevorzugt kontinuierlich extrahiert. Für das erfindungsgemäße Verfahren ist auch eine unvollständige Extraktion der Produkte aus der wässrigen Reaktionsmischung ausreichend.

Nach dem erfindungsgemäßen Verfahren ist bei diskontinuierlicher (batchweiser) Extraktion gegenüber den aus dem Stand der Technik bekannten 2-Phasen-Verfahren eine weitere, durch Zusatz eines organischen Lösemittels erhaltene, mit dem wässrigen Reaktionsmedium nicht mischbare Phase erst in einem zweiten Schritt nach erfolgter Reduktion zum Zwecke der Extraktion der gewünschten Reaktionsprodukte zugegen.

Bei der kontinuierlichen Extraktion wird nach dem erfindungsgemäßen Verfahren das wässrige Reaktionsmedium eines oder mehrerer Reaktionsgefäße in einer separaten, angeschlossenen Extraktionsanlage mit der organischen Extraktionsphase kontaktiert. Vorzugsweise wird lediglich ein Teil des Reaktionsmediums extrahiert. In einer besonders bevorzugten Ausführungsform wird das Reaktionsmedium in einem separaten Anlagenteil mit der Extraktionsphase portionsweise und im gleichmäßigen Strom kontaktiert und wieder getrennt (Entmischung).

In einer möglichen Ausführungsform der kontinuierlichen Extraktion im gleichmäßigen Strom wird eine Extraktionskolonne verwendet, in der die wässrige Phase und die Extraktionsphase im Gegenstrom in einer vertikalen Säule, vorzugsweise einer Glassäule kontaktiert werden, die Siebböden enthält, welche zur Verwirbelung und/ oder Durchmischung der Phasen führen.

Die kontinuierliche Extraktion ist insbesondere dann zu bevorzugen, falls das erfindungsgemäße Verfahren über einen langen Zeitraum kontinuierlich betrieben werden soll.

In einer besonders bevorzugten Ausführungsform wird in mehreren Reaktionsgefäßen parallel die Reduktion in der wässrigen Phase gemäß dem Schritt a) durchgeführt und anschließend aus einem oder mehreren Gefäßen ganz oder teilweise das wässrige Reaktionsmedium entnommen und auf die oben beschriebene Art und Weise separat extrahiert. Die extrahierte wässrige Phase kann anschließend sofort weiter umgesetzt werden, während noch nicht extrahierte Anteile kontinuierlich der Extraktion und anschließend einem anderen Gefäß zugeführt werden. Auf diese Weise können mehrere Reaktionsansätze zeitversetzt und in mehreren Reaktionsgefäßen parallel realisiert werden. Auf diese Weise werden auch gleichzeitig ungenutzte Standzeiten des wässrigen enzymhaltigen Reaktionsmediums vermieden. Folglich ist lediglich derjenige Teil des wässrigen Reaktionsmediums, der gerade Schritt b) (Extraktion) durchläuft, vorübergehend nicht produktiv.

Bevorzugt wird bei der Extraktion unabhängig davon, ob diese diskontinuierlich oder kontinuierlich ausgeführt wird, eine möglichst kurze Kontaktzeit zwischen wässriger Phase (Reaktionsmedium) und organischer Extraktionsphase angestrebt. Besonders bevorzugt sind Kontaktzeiten zwischen 1min und 60min, insbesondere von 1min bis 10min.

Als organische Lösemittel sind alle mit Wasser nicht mischbaren Lösemittel geeignet, die den gebildeten sekundären Alkohol aus der wässrigen Phase isolieren können.

Bevorzugt werden organische Lösemittel ausgewählt aus der Gruppe der Ester und/oder Ether und/oder Alkane verwendet.

Besonders bevorzugt werden Ethylacetat, Methylacetat, Propylacetat, Isopropylacetat, Butylacetat, Tertbutylacetat, Diethylether, Diisopropylether, Dibutylether, Tertbutylmethylether (MTBE), n-Pentan, n-Hexan und n-Heptan oder deren Mischungen verwendet. , Ganz besonders bevorzugt wird MTBE verwendet.

Nach der Abtrennung der organischen Extraktionsphase wird diese vorzugsweise destillativ aufgearbeitet, wobei eine Anreicherung des Reaktionsproduktes erreicht und die teilweise bis vollständige Abtrennung von Nebenprodukten vom Extraktionslösemittel bewirkt wird und dieses erneut zur Extraktion eingesetzt werden kann.

Das destillativ aufbereitete organische Lösemittel kann für den wiederholten bzw. kontinuierlichen Extraktionseinsatz verwendet werden.

Die nach der Extraktion verbleibende wässrige Phase wird im erfindungsgemäßen Verfahren erneut mit Edukt der allgemeinen Formel (I) (Ketoverbindung) und Reduktionsmittel versetzt und inkubiert. Dabei können vor der Inkubation erforderlichenfalls zusätzlich Enzym und Coenzym zugesetzt werden.

Die Reduktion wird bei vermindertem Druck durchgeführt, im allgemeinen bei einem Druck von 1 mbar bis <1 bar, bevorzugt 1 mbar bis 100 mbar, besonders bevorzugt von 30 mbar bis 70 mbar, und die dabei leicht flüchtigen Bestandteile kontinuierlich aus dem Reaktionssystem entfernt.

Insbesondere eignet sich diese Vorgehensweise beim Einsatz von Isopropanol als Reduktionsmittel und der kontinuierlichen Entfernung der leicht flüchtigen Bestandteile umfassend Aceton aus der Reaktionsmischung.

Auf diese Weise wird überraschenderweise eine besonders gute Extraktionsleistung und Wiederverwendbarkeit der Extraktionsphase erreicht.

Durch Aufreinigung der organischen Extraktionslösung enthaltend das Rohprodukt, beispielsweise mittels Feindestillation, erhält man das gewünschte Endprodukt. Die so erhaltenen Produkte zeichnen sich typischerweise durch Ausbeuten >95 % und ee >99 % aus.

Das erfindungsgemäße Verfahren ermöglicht den Wiedereinsatz eines wässrigen, enzymhaltigen Reaktionsmediums zur Umsetzung von Ketonen zu chiralen Alkoholen. Die sich ergebenden hohen Raum-Zeit-Leistungen und der durch wiederholten Einsatz der Enzymlösung geringe Enzymverbrauch pro Mol Produkt ermöglichen die kostengünstige Umsetzung von Ketonen zu chiralen Alkoholen unter Verwendung von Enzymen.

Zudem erlaubt der Wiedereinsatz auf eine aufwändige vollständige Produktextraktion zu verzichten und stattdessen eine weniger aufwendige nur teilweise Extraktion durchzuführen. Ferner kann damit die zu entsorgende wässrige Phase drastisch verringert werden.

In der besonders bevorzugten Ausführungsform wird zudem eine besonders effiziente Produktextraktion mit geringem Verbrauch an Extraktionslösemittel gewährleistet.

Die aus dem Stand der Technik bekannten Verfahren - unabhängig davon ob einphasige oder zweiphasige Prozesse - zur enzymkatalysierten Herstellung chiraler Alkohole ausgehend von prochiralen Ketonen geben dem Fachmann keinerlei Hinweis darauf, dass die prozessuale Trennung von Reduktion und Extraktion, kombiniert mit einer Rückführung der wässrigen Phase zu signifikanten Vorteilen der geschilderten Art führen.

Die Erfindung wird durch die folgenden Beispiele erläutert:

### Beispiel 1a:

### Umsetzung von Acetessigester in wässriger Phase

### Durchführung

400 mL einer Lösung von Wasser, Phosphatpuffer (50 mM), Isopropanol (1.0 M), 25 U / mL ADH-LB (Rohextrakt), NADP-Dinatriumsalz (0.0415 mM) und Acetessigester (0.5 M) von pH 6,5 wurde in einem 500 mL Rundkolben mit Magnetrührer und Rückflusskühler gefüllt und bei 30°C kräftig gerührt. Nach 19 Stunden wurde die Lösung mit 4 x 400mL Methyl-tertbutylether (MTBE) extrahiert und das Produkt durch eindampfen der organischen Phasen isoliert. Mittels GC- und NMR-Spektroskopie wurde die Ausbeute an *(R)*-3-Hydroxy-butansäureethylester in der Auswage bestimmt.

### Ergebnis

- Produkt-Ausbeute: 190 mmol (95%)
- Raum-Zeit-Leistung: 25 Mol/m³h

### Beispiel 1b:

### Wiedereinsatz der extrahierten wässrigen Phase

### Durchführung

Die nach Extraktion mit MTBE zurückbleibende Wasserphase aus Beispiel 1a wurde mit Isopropanol (400 mmol) und Acetessigester (200 mmol) versetzt und nach Einstellen auf pH 6,5 in einen 500 mL Rundkolben mit Magnetrührer und Rückflusskühler gefüllt und bei 30°C kräftig gerührt.
Nach 19 Stunden wurde die Lösung mit 4 x 400mL MTBE extrahiert und das Produkt durch eindampfen der organischen Phasen isoliert. Mittels GC- und NMR-Spektroskopie wurde die Ausbeute an *(R)*-3-Hydroxy-butansäureethylester in der Auswage bestimmt.

### Ergebnis

- Produkt-Ausbeute: 190 mmol (95%)
- Raum-Zeit-Leistung: 25 Mol/m³h

### Vergleichsbeispiel 2a:

### Umsetzung von Acetessigester im zweiphasigen Gemisch

### Durchführung

200 mL einer Lösung von Wasser, Phosphatpuffer (50 mM), Isopropanol (1.0 M), 25 U / mL ADH-LB (Rohextrakt), NADP-Dinatriumsalz (0.0415 mM) und Acetessigester (0.5 M) von pH 6.5 wurde mit 200 mL MTBE versetzt und in einen 500 mL Rundkolben mit Magnetrührer und Rückflusskühler gefüllt und bei 30°C kräftig gerührt. Nach 19 Stunden wurde die organische Phase abgetrennt, die wässrige Lösung mit 3 x 200mL MTBE extrahiert und das Produkt durch eindampfen der organischen Phasen isoliert. Mittels GC-und NMR-Spektroskopie wurde die Ausbeute an *(R)*-3-Hydroxy-butansäureethylester in der Auswage bestimmt.

### Ergebnis

- Produkt-Ausbeute: 83 mmol (83%)
- Raum-Zeit-Leistung: 10,9 Mol/m³h

### Vergleichsbeispiel 2b:

### Wiedereinsatz der extrahierten wässrigen Phase

### Durchführung

Die nach Extraktion mit MTBE zurückbleibende Wasserphase aus Beispiel 2a wurde mit Isopropanol (200 mmol) und Acetessigester (100 mmol) versetzt und nach Einstellen auf pH 6,5 mit 200 mL MTBE versetzt und in einen 500 mL Rundkolben mit Magnetrührer und Rückflusskühler gefüllt und bei 30°C kräftig gerührt. Nach 19 Stunden wurde die organische Phase abgetrennt, die wässrige Lösung mit 3 x 200mL MTBE extrahiert und das Produkt durch eindampfen der organischen Phasen isoliert. Mittels GC-und NMR-Spektroskopie wurde die Ausbeute an *(R)*-3-Hydroxy-butansäureethylester in der Auswage bestimmt.

### Ergebnis

- Produkt-Ausbeute: 85 mmol (85%)
- Raum-Zeit-Leistung: 11,2 Mol/m³h

**Tabelle 1:**

| | **Beispiel 1a** 1-phasig / Erstansatz | **Beispiel 1b** 1-phasig / Wiedereinsatz | **Vergleichsbeispiel 2a** 2-phasig / Erstansatz | **Vergleichsbeispiel 2b** 2-phasig / Wiedereinsatz |
|---|---|---|---|---|
| Ausbeute | **95%** | **95%** | **83%** | **85%** |
| **Raum-Zeit-Leistung** | 25 Mol/m³h | 25 Mol/m³h | 10,9 Mol/m³h | 11,2 Mol/m³h |

Das Einphasensystem zeigt sowohl beim Erstansatz als auch beim Wiedereinsatz der extrahierten Wasserphase einen besseren Substratumsatz und eine deutlich höhere Raum-Zeit-Leistung als das durch Zusatz von MTBE erhaltene Zweiphasensystem.

### Beispiel 3:

### Enzymkatalysierte Herstellung von (R)-3-Hydroxy-butansäureethylester im 100L Maßstab unter Wiedereinsatz der enzymhaltigen wässrigen Phase

Für die enzymkatalysierte Synthese von *(R)*-3-Hydroxy-butansäureethylester wurde rekombinante Alkoholdehydrogenase aus *Lactobacillus brevis* (=ADH-LB) als Rohextrakt mit einer durchschnittlichen Volumenaktivität von 1.08 kU/mL und als Coenzym β-NADP-Dinatriumsalz (97% chem. Reinheit) eingesetzt.

### 3A. Erstansatz

**Tabelle 2:**

| **Substanz** | **Menge bzw. Volumen** |
|---|---|
| Wasser | 84 L |
| NaCl | 0,84 kg |
| KOH* | ca. 0,39 kg |
| Phosphorsäure (85%ig) | 0,29 L |
| MgCl₂ x 6H₂O | 17,1 g |
| iso-Propanol | 7,65 L |
| Ethylacetoacetat | 6,51 kg |
| NADP Dinatriumsalz | 3,27 g |
| ADH-LB (1.08kU/mL) | 2,3 L |

| | |
|---|---|
| *zur Einstellung von pH6.5, daher keine exakte Mengenangabe | |

Das Gesamtvolumen der wässrigen Mischung betrug ca. 100L.

In einem emaillierten Kessel mit Rührwerk wurde der pH-Wert der Lösung von Magnesiumchlorid, Natriumchlorid, Phosphorsäure und Wasser durch Zugabe von KOH auf den Wert 6.5 eingestellt und anschließend das NADP-Salz und der Enzymrohextrakt zugegeben. Nach Erwärmen der Mischung auf 30°C wurde das Ethylacetoacetat und Isopropanol zugegeben.

Die Mischung wurde ca. 17h lang gerührt bis der Umsatz laut GC- bzw. NMR-Analyse >97% war, wobei die Temperatur der Mischung zwischen 20 und 30 °C gehalten wurde und der pH-Wert ständig kontrolliert wurde.

Die wässrige Phase wurde mit 400L Tertbutylmethylether (MTBE) im Gegenstrom kontinuierlich extrahiert (Flussrate Extraktionsphase (organische Phase) / Flussrate Reaktionsmedium (wässrige Phase) ca. 4 / 1) und die extrahierte wässrige Phase wurde zum Wiedereinsatz in den Reaktionskessel rückgeführt. Die organische Phase wurde dabei kontinuierlich redestilliert, sodass isolierter *(R)*-3-Hydroxy-butansäureethylester vollständig im Destillationssumpf verblieb. Die Phasentrennung bei der Extraktion erfolgte augenblicklich und vollständig.

### 3B. Erster Wiedereinsatz der wässrigen Phase

Die extrahierte wässrige Lösung wurde mit 7,65L Isopropanol und 6,51 kg Ethylacetat versetzt. Die Mischung wurde mit ADH-LB-Rohextrakt aufgestockt. In diesem Beispiel wurde durchwegs mit 10% der Startmenge, also mit jeweils etwa 25kU ADH-LB aufgestockt. Ebenso wurde zum Wiedereinsatz in diesem Beispiel durchwegs je 10% der Startmenge, also 0,327g NADP-Dinatriumsalz zugegeben. Der pH wurde bei Bedarf durch Zugabe von KOH auf den Wert 6.5 eingestellt.

Die Durchführung der Reaktion erfolgte analog zu Beispiel 3A.

Die wässrige Lösung wurde analog Beispiel 3A mit redestilliertem MTBE aus Beispiel 3A extrahiert.

### 3C. Zweiter Wiedereinsatz der wässrigen Phase

Die extrahierte wässrige Lösung aus Beispiel 3B wurde analog zu Beispiel 3B aufgestockt und analog zu Beispiel 3A umgesetzt.

Da die Phasentrenngeschwindigkeit erfahrungsgemäß nach wiederholtem Einsatz des redestillierten MTBE nachlässt, wurde diese Extraktionsphase nach destillativer Aufarbeitung und Abtrennung des Produktes verworfen und durch 400L frisches MTBE ersetzt.

### 3D. Dritter bis fünfter Wiedereinsatz der wässrigen Phase

Die Durchführung der Reaktionen erfolgte analog zu den Beispielen 3B und 3C.

Die Durchführung der Extraktionen erfolgte analog zu den Beispielen 3A bis 3C.

Bei nachlassender Phasentrenngeschwindigkeit wurde die redestillierte organische Extraktionsphase jeweils analog durch frisches MTBE ersetzt.

**Tabelle 3 (Zusammenfassung der Ergebnisse aus den Beispielen 3A bis 3D) :**

| **Beispiel 3** | **Zeit (h)** | **Umsatz (%)** | **ee** |
|---|---|---|---|
| **Erstansatz (3A)** | 17 | 99 | >99 % |
| **1. Wiedereinsatz (3B)** | 17 | 98 | >99 % |
| **2. Wiedereinsatz (3C)** | 16 | 97 | >99 % |
| **3. Wiedereinsatz (3D/1)** | 17 | 98 | >99 % |
| **4. Wiedereinsatz (3D/2)** | 17 | 98 | >99 % |
| **5. Wiedereinsatz (3D/3)** | 17 | 98 | >99 % |

### 3E. Destillation des Rohprodukts

Nach vollständiger Abtrennung von MTBE und weitgehender Entfernung des Isopropanols konnten durch fraktionierte Destillation 38 kg (96%) *(R)*-3-Hydroxy-butansäureethylester mit einer-chemischen Reinheit von >99% und einer optischen Reinheit von >99% ee erhalten werden.

**Tabelle 4:**

| **Beispiel 3** | | |
|---|---|---|
| Substrateinsatz: | | 39,06 kg (300 Mol) |
| Ausbeute: | 96 % | 38.02kg (288 Mol) |
| Raum-Zeit-Leistung: | 28.5 Mol/m³h | 684 Mol/m³d |
| Enzymverbrauch: | 1,25 MU | 4,3 kU/mol-Produkt |
| Coenzymverbrauch: | 6,23 mmol | 17 mg/mol-Produkt |

### Beispiel 4 (erfinderisches Verfahren):

### Enzymkatalysierte Herstellung von (R)-3-Hydroxy-butansäureethylester unter Wiedereinsatz der enzymhaltigen wässrigen Phase bei vermindertem Druck

### 4A. Ersteinsatz

Es wurden die gleichen Reagenzien in gleicher Menge bzw. Volumen eingesetzt wie in Beispiel 3A (siehe Tabelle 2).

Die Reaktionsmischung wurde analog zu Beispiel 3A hergestellt.

Nach Verschließen des Reaktionskessels wurde im Innenraum über der Reaktionsmischung durch Abpumpen ein Druck von ca. 60 mbar eingestellt. Die Mischung wurde ca. 17h lang gerührt bis der Umsatz laut GC- bzw. NMR-Analyse >97% war, wobei die Temperatur der Mischung zwischen 20 und 30 °C gehalten wurde und der pH-Wert ständig kontrolliert wurde.

Die wässrige Lösung wurde mit 400L Tertbutylmethylether (MTBE) im Gegenstrom kontinuierlich extrahiert (Flussrate Extraktionsphase (organische Phase) / Flussrate Reaktionsmedium (wässrige Phase) ca. 4 / 1).
Die organische Phase wurde dabei kontinuierlich redestilliert, sodass isolierter *(R)*-3-Hydroxy-butansäureethylester vollständig im Destillationssumpf verblieb. Die extrahierte Wasserphase wurde zum Wiedereinsatz in den Reaktionskessel rückgeführt. Die Phasentrennung bei der Extraktion erfolgte augenblicklich und vollständig.

### 4B. Wiedereinsatz der wässrigen Phase

Die extrahierte wässrige Lösung wurde mit 7,65L Isopropanol und 6,51 kg Ethylacetat versetzt. Die Mischung wurde mit ADH-LB-Rohextrakt aufgestockt. In diesem Beispiel wurde durchwegs mit 10% der Startmenge, also mit jeweils etwa 25kU ADH-LB aufgestockt. Ebenso wurde zum Wiedereinsatz in diesem Beispiel durchwegs je 10% der Startmenge, also 0,327g NADP-Dinatriumsalz zugegeben. Der pH wurde bei Bedarf durch Zugabe von KOH auf den Wert 6.5 eingestellt.

Die Durchführung der Reaktion erfolgte analog zu Beispiel 4A. Die Aufarbeitung erfolgte analog zu Beispiel 4A. Der Wiedereinsatz der wässrigen Phase wurde analog insgesamt 5 mal ausgeführt (Beispiele 4B/a - 4B/e).

Bei der Durchführung des erfindungsgemäßen Verfahrens unter vermindertem Druck wird der Acetongehalt des MTBE unter der kritischen Grenze gehalten, und die Phasentrennung bei den Extraktionen erfolgte jeweils augenblicklich und vollständig. Die Extraktionsphase kann auf diese Weise beliebig oft wiederverwendet werden.

**Tabelle 5 (Zusammenfassung der Ergebnisse aus den Beispielen 4A und 4B/a bis 4B/e) :**

| **Beispiel 4** | **Zeit (h)** | **Umsatz (%)** | **ee** |
|---|---|---|---|
| **Erstansatz (4A)** | 17 | 99 | >99 % |
| **1. Wiedereinsatz (4B/a)** | 17 | 98 | >99 % |
| **2. Wiedereinsatz (4B/b)** | 16 | 97 | >99 % |
| **3. Wiedereinsatz (4B/c)** | 17 | 98 | >99 % |
| **4. Wiedereinsatz (4B/d)** | 17 | 98 | >99 % |
| **5. Wiedereinsatz (4B/e)** | 17 | 99 | >99 % |

### 4C. Destillation des Rohprodukts

Nach vollständiger Abtrennung von MTBE und weitgehender Entfernung des Isopropanols konnten durch fraktionierte Destillation 38,4kg (97%) *(R)*-3-Hydroxy-butansäureethylester mit einer chemischen Reinheit von >99% und einer optischen Reinheit von >99% ee erhalten werden.

**Tabelle 6:**

| **Beispiel 4** | | |
|---|---|---|
| Substrateinsatz: | | 39,06 kg (300 Mol) |
| Ausbeute: | 97 % | 38.42kg (291 Mol) |
| Raum-Zeit-Leistung: | 28.8 Mol/m³h | 691 Mol/m³d |
| Enzymverbrauch: | 1,25 MU | 4,3 kU/mol-Produkt |
| Coenzymverbrauch: | 6,23 mmol | 17 mg/mol-Produkt |

| | | |
|---|---|---|
| • Der Wiedereinsatz der extrahierten Wasserphase lieferte konstant hohe Umsätze und Enantiomerenüberschüsse. • Das redestillierte Extraktionslösemittel konnte uneingeschränkt wiedereingesetzt werden. | | |

**Tabelle 7:**

| | **Beispiel 3** | **Beispiel 4** |
|---|---|---|
| **Umsatz der Wasserphase bei vermindertem Druck** | nein | ja |
| **Wiedereinsetzbarkeit des Extraktionslösemittels** | 2 x | unbeschränkt |

### Beispiel 5:

### Enzymkatalysierte Herstellung von (S)-2-Hexanol im 4000L Maßstab unter Wiedereinsatz der enzymhaltigen wässrigen Phase

Für die enzymkatalysierte Synthese von (S)-2-Hexanol wurde rekombinante Alkoholdehydrogenase aus Thermoanaerobium spec. (= ADH-T) als Rohextrakt mit einer durchschnittlichen Volumenaktivität von 545 U/mL und als Coenzym β-NADP-Dinatriumsalz (97% chem. Reinheit) eingesetzt.

### 5A. Erstansatz

**Tabelle 8:**

| **Substanz** | **Menge bzw. Volumen** |
|---|---|
| Wasser | 2960 L |
| NaOH* | ca. 9.8 kg* |
| Phosphorsäure (85%ig) | 17.6 kg |
| MgCl2 x 6H2O | 600 g |
| iso-Propanol | 800 L |
| 2-Hexanon | 199.5 kg |
| NADP Dinatriumsalz | 160 g |
| ADH-T (545U/mL) | 22 L |

| | |
|---|---|
| *zur Einstellung von pH 7.0, daher keine exakte Mengenangabe | |

Das Gesamtvolumen der wässrigen Mischung betrug ca. 4000L.

In einem emaillierten 8000L Kessel mit Rührwerk wurde der pH-Wert der Lösung von Magnesiumchlorid, Phosphorsäure und Wasser durch Zugabe von NaOH auf den Wert 6,5 eingestellt und anschließend 2-Hexanon, Isopropanol, NADP-Salz und der Enzymrohextrakt zugegeben. Nach Einstellen von pH 7,0 durch Zugabe von NaOH wurde das Gemisch auf 30 °C erwärmt.

Die Mischung wurde so lange gerührt bis der Umsatz laut GC-Analyse 74% war.

Die wässrige Phase wurde zweimal mit n-Heptan (600L und 400L) extrahiert und die extrahierte wässrige Phase wurde zum Wiedereinsatz in den Reaktionskessel rückgeführt und unter Vakuum (<100 mbar) und Einblasen von Stickstoff für mehrere Stunden gerührt. Der organische Extrakt wurde zwischengelagert.

### 5B. Wiedereinsatz der wässrigen Phase

Die extrahierte wässrige Lösung wurde mit 573L Isopropanol und 160kg 2-Hexanon versetzt. Der pH wurde mit NaOH auf den Wert 7.0, die Temperatur auf 30 °C eingestellt.

Die Durchführung der Reaktion erfolgte analog zu Beispiel 5A. Die Mischung wurde so lange gerührt bis der Umsatz laut GC-Analyse 70% war.

Die anschließende Extraktion erfolgte analog zu Beispiel 5A.

### 5C. Aufreinigung des Heptanextraktes

Die vereinigten Heptanextrakte wurden nach dem Auskreisen von Wasser aufkonzentriert und anschließend fraktionierend destilliert.
Dabei konnten insgesamt 216kg (59% d. Th.) (S)-2-Hexanol von >99 %ee erhalten werden.

### Tabellen 9+10 (Zusammenfassung der Ergebnisse aus den Beispielen 5A bis 5C):

| **Reaktion** | **Umsatz (%)** | **ee** |
|---|---|---|
| **Erstansatz (5A)** | 74 | >99 % |
| **Wiedereinsatz (5B)** | 70 | >99 % |

| **Destillation (5C)** | **Ausbeute** | **% d.Th.** | **ee** |
|---|---|---|---|
| **(*S*)-2-Hexanol** | 216kg | 59 | >99 % |

### Beispiel 6:

### Enzymkatalysierte Herstellung von (S)-2-Pentanol im 3000L Maßstab

Für die enzymkatalysierte Synthese von (S)-2-Pentanol wurde rekombinante Alkoholdehydrogenase aus *Thermoanaerobium* spec. (= ADH-T) als Rohextrakt mit einer durchschnittlichen Volumenaktivität von 545 U/mL und als Coenzym β-NADP-Dinatriumsalz (97% chem. Reinheit) eingesetzt.

### 6A. Erstansatz

**Tabelle 11:**

| **Substanz** | **Menge bzw. Volumen** |
|---|---|
| Wasser | 1200 L |
| NaOH (aq., 25% w/v)* | ca. 10L* |
| Phosphorsäure (85%ig) | 5 kg |
| MgCl₂ x 6H₂O | 183 g |
| iso-Propanol | 1350 L |
| 2-Pentanon | 175 kg |
| NADP Dinatriumsalz | 106 g |
| ADH-T (545U/mL) | 14,7 L |

| | |
|---|---|
| *zur Einstellung von pH7.0, daher keine exakte Mengenangabe | |

Das Gesamtvolumen der wässrigen Mischung betrug ca. 2700L. In einem emaillierten 5000L Kessel mit Rührwerk wurde eine Lösung von Magnesiumchlorid, Phosphorsäure, Wasser und NaOH von pH 6,5 mit 2-Pentanon, Isopropanol, NADP-Salz und der Enzymrohextrakt-Lösung versetzt. Bei pH 7,0 wurde das Gemisch auf 40 °C erwärmt.

Die Mischung wurde so lange gerührt bis der Umsatz laut GC-Analyse 66% war.

Die wässrige Phase wurde zweimal mit n-Pentan (1000L und 300L) extrahiert und die extrahierte wässrige Phase wurde zum Wiedereinsatz in den Reaktionskessel rückgeführt und unter Vakuum (<100 mbar) und Einblasen von Stickstoff für mehrere Stunden gerührt. Der organische Extrakt wurde zwischengelagert.

### 6B. Wiedereinsatz der wässrigen Phase

Die extrahierte wässrige Lösung wurde mit 1350L Isopropanol und 175kg 2-Pentanon versetzt. Der pH wurde mit NaOH auf den Wert 7.0, die Temperatur auf 40 °C eingestellt.

Die Durchführung der Reaktion erfolgte analog zu Beispiel 6A. Die Mischung wurde so lange gerührt bis der Umsatz laut GC-Analyse 64% war. Die anschließende Extraktion erfolgte analog zu Beispiel 6A.

### 6C. Aufreinigung des Pentanextraktes

Die vereinigten Pentanextrakte wurden nach dem Auskreisen von Wasser aufkonzentriert und anschließend fraktionierend destilliert.

Dabei konnten insgesamt 208 kg (58% d. Th.) (S)-2-Pentanol von >99 %ee erhalten werden.

### Tabellen 12+13 (Zusammenfassung der Ergebnisse aus Beispiel 6):

| **Reaktion** | **Umsatz (%)** | **ee** |
|---|---|---|
| **Erstansatz (6A)** | 66 | >99 % |
| **Wiedereinsatz (6B)** | 64 | >99 % |

| **Destillation (6C)** | **Ausbeute** | **% d.Th.** | **ee** |
|---|---|---|---|
| **(*S*)-2-Pentanol** | 208 kg | 58 | >99 % |

## Patentansprüche

1. Verfahren zur Herstellung chiraler sekundäre Alkohole enthaltend die Schritte
a) Reduktion einer Ketoverbindung in einem wässrigen Reaktionsmedium, enthaltend Wasser, Reduktionsmittel, Alkohol-Dehydrogenase und Coenzym unter vermindertem Druck und Entfernung leicht flüchtiger Bestandteile aus dem Reaktionssystem,
b) Extraktion des gebildeten sekundären Alkohols mittels einer weiteren Phase, enthaltend ein nicht mit Wasser mischbares organisches Lösemittel unter Verwirbelung und/oder Durchmischung der wässerigen und organischen Phasen,
c) nach Entmischung der Phasen, Abtrennung der zur Extraktion verwendeten Phase und Wiederverwendung des wässrigen Reaktionsmediums in Schritt a).

2. Verfahren nach Anspruch 1, **dadurch gekennzeichnet, dass** als Reduktionsmittel Isopropanol eingesetzt wird.

3. Verfahren nach Anspruch 1, **dadurch gekennzeichnet, dass** als Reduktionsmittel Ameisensäure oder ein Salz der Ameisensäure eingesetzt wird.

4. Verfahren nach einem oder mehreren der Ansprüche 1 bis 3, **dadurch gekennzeichnet, dass** die zur Extraktion verwendete Phase Methyltertbutylether ist.

5. Verfahren nach einem oder mehreren der Ansprüche 1 bis 4, **dadurch gekennzeichnet, dass** die Kontaktzeit zwischen wässrigem Reaktionsmedium und organischer Extraktionsphase bei der Extraktion 1 min bis 10 min beträgt.

6. Verfahren nach einem oder mehreren der Ansprüche 1 bis 5, **dadurch gekennzeichnet, dass** Alkohol-Dehydrogenasen aus Hefe, Pferdeleber, Rhodococcus erythropolis, Thermoanaerobium spec., Lactobacillus kefir oder Lactobacillus brevis eingesetzt werden.

7. Verfahren nach einem oder mehreren der Ansprüche 1 bis 6, **dadurch gekennzeichnet, dass** als Coenzyme NADP, NADPH, NAD, NADH oder deren Salze eingesetzt werden.

8. Verfahren nach einem oder mehreren der Ansprüche 1 bis 7, **dadurch gekennzeichnet, dass** als Ketoverbindungen prochirale Ketone der allgemeinen Formel (I)
R¹-C(O)-R² (I)
eingesetzt werden, wobei
**R¹** und **R²** unabhängig voneinander ausgewählt werden aus der Gruppe enthaltend C₁-C₂₀-Alkyl, C₃-C₂₀-Cycloalkyl, C₅-C₂₀-Aryl, C₁-C₂₀-Heteroaryl, C₂-C₂₀-Alkenyl, C₅-C₂₀-Aralkyl, C₅-C₂₀-Alkylaryl oder R¹ und R² zusammen einen Ring bilden können
und **R¹** und **R²** gegebenenfalls unabhängig voneinander mit einem oder mehreren Resten **Z** substituiert sein können, wobei
**Z** ausgewählt wird aus der Gruppe enthaltend Fluor, Chlor, Brom, Iod, - CN, -NO₂, -NO, -NR³OR³, -CHO, -SO₃H, -COOH oder -R³ und
**R³** für Wasserstoff steht oder die Bedeutung von **R¹** haben kann und
in R¹ und R² gegebenenfalls unabhängig voneinander eine oder mehrere Methylengruppen durch gleiche oder verschiedene Gruppen Y ersetzt sein können, wobei
**Y** ausgewählt wird aus der Gruppe enthaltend -CR³=CR³-, -C≡C-, -C(O)-, - C(O)O-, -OC(O)-, -C(O)OC(O)-, -O-, -O-O-, -CR³=N-, - C (O) -NR³-, -N=N-, - NR³-NR³-, -NR³-O-, -NR³-, -P (O) (OR³) O-, -OP(O) (R³) O-, -P (R³) -, -P(O) (R³)-, -S-, -S-S-, -S(O)-, -S (O)₂-, - S(O)NR³-, -S(O)(OR³)O-, -Si(R³)₂-, -Si(R³)₂O-, -Si(R³)(OR³)-, -OSi(R³)₂O-, -OSi(R³)₂-oder -Si(R³)₂OSi(R³)₂-.

## Claims

1. Process for preparing chiral secondary alcohols, comprising the steps of
a) reducing a keto compound in an aqueous reaction medium containing water, reducing agent, alcohol dehydrogenase (ADH) and coenzyme under reduced pressure and removing volatile constituents from the reaction system,
b) extracting the secondary alcohol formed by means of a further phase containing a water-immiscible organic solvent, with vortexing and/or mixing of the aqueous and organic phases,
c) after separation of the phases, removing the phase used for extraction and reusing the aqueous reaction medium in step a).

2. Process according to claim 1, **characterized in that** the reducing agent used is isopropanol.

3. Process according to claim 1, **characterized in that** the reducing agent used is formic acid or a salt of formic acid.

4. Process according to one or more of claims 1 to 3, **characterized in that** the phase used for extraction is methyl tert-butyl ether.

5. Process according to one or more of claims 1 to 4, **characterized in that** the time of contact between the aqueous reaction medium and the organic extraction phase during extraction is from 1 min to 10 min.

6. Process according to one or more of claims 1 to 5, **characterized in that** alcohol dehydrogenases from yeast, equine liver, Rhodococcus erythropolis, Thermoanaerobium spec., Lactobacillus kefir or Lactobacillus brevis are used.

7. Process according to one or more of claims 1 to 6, **characterized in that** the coenzymes used are NADP, NADPH, NAD, NADH or salts thereof.

8. Process according to one or more of claims 1 to 7, **characterized in that** the keto compounds used are prochiral ketones of the general formula (I)
R¹-C(O)-R² (I),
in which
**R¹** and **R²** are selected independently of one another from the group comprising C₁-C₂₀-alkyl, C₃-C₂₀-cycloalkyl, C₅-C₂₀-aryl, C₁-C₂₀-heteroaryl, C₂-C₂₀-alkenyl, C₅-C₂₀-aralkyl, C₅-C₂₀-alkylaryl or R¹ and R² together can form a ring
and **R¹** and **R²** may, where appropriate independently of one another, be substituted with one or more radicals **Z,** where
**Z** is selected from the group comprising fluoro, chloro, bromo, iodo, -CN, -NO₂, -NO, -NR³OR³, -CHO, -SO₃H, -COOH or -R³ and
**R³** is hydrogen or may have the meaning of R¹, and
in R¹ and R², where appropriate independently of one another, one or more methylene groups may be replaced by identical or different groups **Y**, where
**Y** is selected from the group comprising -CR³=CR³-, -C≡C-, -C(O)-, -C(O)O-, -OC(O)-, -C(O)OC(O)-, -O-, -O-O-, -CR³=N-, -C(O)-NR³-, -N=N-, -NR³-NR³-, -NR³-O-, -NR³-, -P(O)(OR³)O-, -OP(O)(R³)O-, -P(R³)-, -P(O)(R³)-, -S-, -S-S-, -S(O)-, -S(O)₂-, -S(O)NR³-, -S(O)(OR³)O-, -Si(R³)₂-, -Si(R³)₂O-, -Si(R³)(OR³)-, -OSi(R³)₂O-, -OSi(R³)₂- or -Si(R³)₂OSi(R³)₂-.

## Revendications

1. Procédé de préparation d'alcools secondaires chiraux contenant les étapes :
a) réduction d'un composé cétonique dans un milieu réactionnel aqueux contenant de l'eau, un réducteur, une alcool-déhydrogénase et une co-enzyme, sous pression réduite, et élimination, du système réactionnel, des constituants facilement volatils,
b) extraction de l'alcool secondaire formé, à l'aide d'une phase supplémentaire contenant un solvant organique non miscible à l'eau, en présence d'une turbulence et/ou d'une opération de mélange intime de la phase aqueuse et de la phase organique,
c) après démixion des phases, séparation de la phase utilisée pour l'extraction, et réutilisation du milieu réactionnel aqueux dans l'étape a).

2. Procédé selon la revendication 1, **caractérisé en ce qu'**on utilise en tant que réducteur l'isopropanol.

3. Procédé selon la revendication 1, **caractérisé en ce qu'**on utilise en tant que réducteur l'acide formique ou un sel de l'acide formique.

4. Procédé selon l'une ou plusieurs des revendications 1 à 3, **caractérisé en ce que** la phase utilisée pour l'extraction est le méthyl-tert-butyl-éther.

5. Procédé selon l'une ou plusieurs des revendications 1 à 4, **caractérisé en ce que** le temps de contact entre le milieu réactionnel aqueux et la phase organique d'extraction est, lors de l'extraction, de 1 minute à 10 minutes.

6. Procédé selon l'une ou plusieurs des revendications 1 à 5, **caractérisé en ce qu'**on utilise les alcool-déhydrogénases de la levure, du foie de cheval, de Rhodococcus erythropolis, de Thermoanaerobium spec., de Lactobacillus kefir ou de Lactobacillus brevis.

7. Procédé selon l'une ou plusieurs des revendications 1 à 6, **caractérisé en ce qu'**on utilise en tant que co-enzyme le NADP, le NADPH, le NAD, le NADH ou leurs sels.

8. Procédé selon l'une ou plusieurs des revendications 1 à 7, **caractérisé en ce qu'**on utilise en tant que composés cétoniques des cétones prochirales de formule générale (I)
R¹-C(O)-R² (I)
dans laquelle
R¹ et R² sont chacun indépendamment de l'autre choisis dans le groupe contenant les radicaux alkyle en C₁-C₂₀, cycloalkyle en C₃-C₂₀, aryle en C₅-C₂₀, hétéroaryle en C₁-C₂₀, alcényle en C₂-C₂₀, aralkyle en C₅-C₂₀, alkylaryle en C₅-C₂₀, ou encore R¹ et R² peuvent ensemble former un cycle,
et R¹ et R² peuvent éventuellement indépendamment l'un de l'autre être substitués par un ou plusieurs radicaux Z, où Z est choisi dans le groupe contenant les radicaux fluoro, chloro, bromo, iodo, -CN, -NO₂, -NO, -NR³OR³, -CHO, -SO₃H, -COOH ou -R³, et
R³ est un atome d'hydrogène ou peut avoir les significations de R¹, et
dans R¹ et R², un ou plusieurs groupes méthylène peuvent éventuellement, indépendamment les uns des autres, être remplacés par des groupes Y identiques ou différents, où
Y est choisi dans le groupe contenant les radicaux -CR³=CR³-, -C(O)-, -C≡C-, -C(O)O-, -OC(O)-, - C(O)OC(O)-, -O-, -O-O-, -CR³=N-, -C(O)-NR³-, -N=N-, -NR³-NR³-, -NR³-O-, -NR³-, -P(O)(OR³)O-, -OP(O)(R³)O-, -P(R³)-, -P(O)(R³)-, -S-, -S-S-, -S(O)-, -S(O)₂-, -S(O)NR³-,-S(O)(OR³)O-, -Si(R³)₂-, -Si(R³)₂O-, -Si(R³)(OR³)-, - OSi(R³)₂O-, -OSi(R³)₂- ou -Si(R³)₂Si(R³)₂-.
